# EUROPÄISCHE PATENTSCHRIFT

(11) **EP 0 805 693 B1**
(45) Veröffentlichungstag und Bekanntmachung des Hinweises auf die Patenterteilung: **08.12.1999**
(21) Anmeldenummer: 96901288.9
(22) Anmeldetag: 17.01.1996
(51) Int. Cl.: A61M 1/00, A61B 5/03

(54) **HALTERUNGSVORRICHTUNG FÜR GEHIRNMESSONDEN**
SECURING DEVICE FOR BRAIN SCAN PROBES
DISPOSITIF DE RETENUE POUR SONDES DE MESURE DE LA PRESSION SANGUINE CEREBRALE

(30) Priorität: 25.01.1995 DE 19502183
(43) Veröffentlichungstag der Anmeldung: 12.11.1997
(73) Patentinhaber: Fleckenstein, Wolfgang, 24247 Mielkendorf (DE)
(72) Erfinder: Fleckenstein, Wolfgang, 24247 Mielkendorf (DE)
(74) Vertreter: Schaefer, Konrad, Dipl.-Phys.
(86) Internationale Anmeldenummer: EP9600178
(87) Internationale Veröffentlichungsnummer: WO9622798

(56) Entgegenhaltungen:
- WO-A-91/12765
- US-A- 4 903 707
- US-A- 5 112 312

## Beschreibung

Die Erfindung betrifft eine Vorrichtung dei im Oberbegriff des Anspruchs 1 genannten Art.

Eine solche Vorrichtung ist aus der US-A-4 903 707 bekannt. Diese bekannte Konstruktion weist einen Einführungskatheter mit distalen Öffnungen auf, dei primär als Dränagekatheter dient und zu diesem Zweck einen äußeren Sauganschluß aufweist, der jedoch auch einen einen distalen Drucksensor aufweisende Sonde aufnimmt, die innerhalb des Einführungskatheters die im Schädel verankerte Schraube durchläuft. An der Durchgangsbohrung der Schraube ist der Einführungskatheter, der zu diesem Zweck hier als starres Rohr ausgebildet ist, mit einer Quetschdichtung steril und zugfest quetschbar.

Bei dieser bekannten Konstruktion ist der Einführungskatheter ausreichend eigensteif, um durch das Gehirngewebe bis zum Verlegeort gezielt vorgeschoben zu werden. Er kann daher auch zur Verlegung dünner hochflexibler Sonden verwendet werden.

Für die Langzeitmessung des Gewebe pO₂, also des Sauerstoffpartialdruckes im Gehirn, ist die bekannte Vorrichtung jedoch nicht verwendbar.

Für diese Zwecke müssen dünne hochflexible Sonden frei im Gehirngewebe angeordnet werden. Die bekannte Konstruktion ist dafür nicht geeignet, da bei ihr die Sonde dauerhaft im Inneren des Einführungskatheters liegen bleibt, wodurch die Messung störend beeinflußt würde.

Der bekannte, aus Gründen der Einführbarkeit sehr steife Einführungskatheter ist außerdem nicht für Langzeitmessungen geeignet, da er bei Bewegungen des Gehirns (Atmung, Pulsschlag) den Bewegungen des empfindlichen Gewebes nicht ausreichend folgen kann und dieses verletzt. Solche Verletzungen sind schädlich für das Gehirn und verfälschen zusätzlich die Sauerstoffmessung.

Außerdem ist die bekannte Vorrichtung für Langzeitmessungen schon deswegen nicht geeignet, weil sie prinzipiell ein offenes System darstellt, das zur Gehirndränage verwendet wird. Der Einführungskatheter stellt daher ständig eine nach außen offene Verbindung dar, die hygienisch bedenklich ist, insbesondere bei sehr langer Meßzeit. Sterile Bedingungen können hier nur unter hoch intensiven Bedingungen gewährleistet werden.

Die Aufgabe der vorliegenden Erfindung besteht darin, eine Vorrichtung der eingangs genannten Art zu schaffen, mit der hochflexible Gewebe-pO₂-Sonden zielgenau in das Gehirn eingeführt und langfristig steril und sicher gehalten werden können.

Diese Aufgabe wird mit den Merkmalen des Anspruchs 1 gelöst.

Bei der erfindungsgemäßen Konstruktion kann der Einführungskatheter ähnlich wie bei der bekannten Konstruktion ausreichend eigensteif ausgebildet sein, um zielgenau zum Meßort in das Gehirngewebe eingeführt zu werden. Bei noch geöffneter Quetschverbindung kann dann die Sonde durch den verlegten Einführungskatheter, in guter Führung in diesem, bis zum Meßort vorgeschoben werden und am Kupplungsstecker in Längsrichtung zugentlastet gesichert mit der Vorrichtung verbunden werden. Anschließend läßt sich der Einführungskatheter mit seiner auf der Sonde längsverschiebbaren Öffnung innerhalb des Schutzschlauclies zurückziehen, bis die Sonde frei im Gehirngewebe liegt. Die Quetschverbindung kann dann geschlossen werden, wobei sie im Gegensatz zur bekannten Konstruktion den quetschbaren Einführungskatheter uni die Sonde abdichtend quetscht. Das Gehirn ist dann an dieser Stelle nach außen völlig hermetisch abgeschlossen, so daß auch langzeitig keine Infektionen zu befürchten sind. Die Sonde liegt nun völlig frei und flexibel den Bewegungen des Gewebes folgend im Gehirn, ohne Beschädigungsgefahr für dieses. Dadurch wird erstmals die Möglichkeit geschaffen, hochflexible Gewebe-pO₂-Sonden zielsicher in das Gehirn einzuführen, anschließend für die Lanzeitmessung jedoch eine freie Lagerung der dünnen Sonde im Gehirn zu gewährleisten.

Vorteilhaft sind die Merkmale des Anspruchs 2 vorgesehen. Auf diese Weise wird die Handhabung der Quetschdichtung wesentlich vereinfacht und erleichtert. Dabei sorgt die Drehsicherung dafür, daß während des Spannens der Quetschdichtung keine Drehkräfte auf den Einführungskatheter ausgeübt werden, dieser also keine das Gehirngewebe zerstörende Drehbewgung ausführt. Die Spanneinrichtung kann daher vorteilhaft als einfache Überwurfschraubmutter ausgebildet sein.

Vorteilhaft sind die Merkmale des Anspruchs 3 vorgesehen. Das Endstück erleichtert die Bedienungsschritte am proximalen Ende des Schutzschlauches. Die aus meßtechnischen Gründen gesondert angelieferte Sonde kann von diesem Ende her eingeführt und sodann mit dem elektrischen Kupplungsstecker befestigt werden. Die axiale Halterung des Einführungskatheters in seiner Einführungsstellung ermöglicht das sichere Einführen des Katheters in das Gehirn, ohne daß dieses in axialer Richtung ausweichen kann.

Vorteilhaft sind die Merkmale des Anspruchs 4 vorgesehen. Mit dem Führungsdraht kann das Einführungskatheter während des Einführens in das Gehirngewebe ausgesteift werden, auch wenn der Katheter selbst nicht aus ausreichend eigensteifem Material besteht.

Vorteilhaft sind die Merkmale des Anspruchs 5 vorgesehen. Auf diese Weise ist es möglich, den Einführungskatheter am proximalen Ende des Schutzschlauches seitlich getrennt von der Sonde zu verlegen. Damit wird die Handhabung erleichtert und es kann der Schutzschlauch durchgehend geschlossen ausgebildet sein.

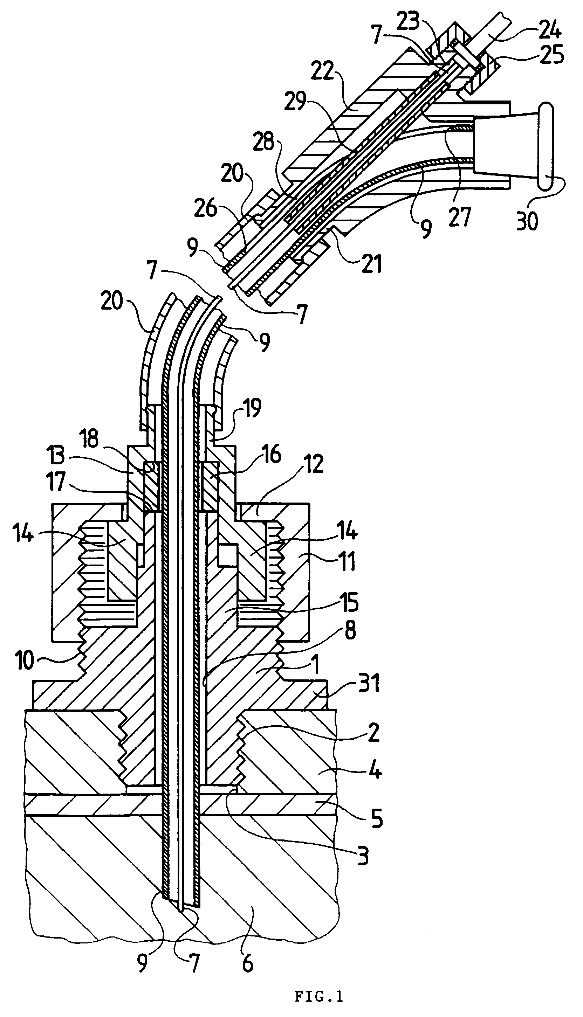

In der Zeichnung ist die Erfindung beispielsweise und schematisch in einem nicht maßstabgetreuen Achsschnitt durch eine Halterungsvorrichtung dargestellt.

Die Figur zeigt eine Schraube 1, die mit einem distalen Außengewinde 2 in eine Schädelbohrung 3 im Schädelknochen 4 eines Patienten eingeschraubt ist. Unter dem Schädelknocken 4 sind die Dura und Gehirngewebe 6 dargestellt.

Eine katheterförmige Sonde 7 ist durch eine Durchgangsbohrung 8 der Schraube 1 und die an dieser Stelle punktierte Dura 5 bis zur dargestellten Tiefe in das Gehirngewebe 6 vorgeschoben. Im Spitzenbereich weist die Sonde 7 eine Meßstelle auf. In der dargestellten, einen Zwischenschritt des Einführungsvorganges zeigenden Darstellung ist die Sonde 7 auf ganzer Länge in das Gehirngewebe 6 hinein von einem Einführungskatheter 9 umgeben, der als Schlauch aus geeignetem Material ausgebildet ist.

Auf dem proximalen Ende der Schraube 1 greift auf ein Außengewinde 10 eine Überwurfmutter 11, die mit einem Innenflansch 12 eine Stufenfläche einer Hülse 13 überfaßt und somit bei Schraubbetätigung die Hülse 13 gegen die Schraube 1 zieht. Die Hülse übergreift mit einer im Querschnitt unsymmetrischen, also drehsichernd wirkenden Schiebeführung 14 einen entsprechenden Ansatz 15 der Schraube 1 und ist somit auf diesem drehgesichert in Achsrichtung verschiebar. An ihrem proximalen Ende bildet die Hülse 13 eine Ringkammer aus, die eine als Schlauchstück ausgebildete Quetschdichtung 16 umgibt. Die Quetschdichtung 16 wird an ihrem Umfang von einer inneren Umfangsfläche der Hülse 13 begrenzt und an ihren Enden von einer Stirnfläche 17 der Schraube 1 und einer Stirnfläche 18 der Hülse 13.

Wird die Überwurfmutter 11 aus der dargestellten Ansatzstellung weiter auf das Außengewinde 10 der Schraube 1 aufgeschraubt, so zieht sie die Hülse 13 gegen die Schraube 1 unter achsialer Verkürzung des Abstandes zwischen den Stirnflächen 17 und 18. Dadurch wird die Quetschdichtung 16, die nach außen nicht ausweichen kann, nach innen gequetscht.

Vorzugsweise ist das Material der Quetschdichtung 16 etwas härter als das des Einführungskatheters 9, so daß eine zugfeste und insbesondere sterile hermetische Abdichtung geschaffen wird zwischen der Schraube 1 und dein Einführungskatheter 9 einerseits und diesem und der Sonde 7 andererseits.

Die Hülse 13 trägt an ihrem proximalen Ende einen Schlauchanschluß 19 zur zugfesten Befestigung eines Schutzschlauches 20, der den Einführungskatheter 9 umgibt.

Der Schutzschlauch 20 ist an seinem proximalen Ende, noch immer den Einführungskatheter 9 mit darin verlaufender Sonde 7 umgebend, auf einem Schlauchstutzen 21 eines Endstückes 22 befestigt, durch welches in einer Bohrung 23 die Sonde 7 nach außen verläuft. Sie ist am äußeren Ende der Bohrung 23 an ihrem elektrischen Kupplungsstecker 24 befestigt, der mit einer Überwurfmutter 25 am Endstück 22 lösbar befestigt ist.

Der Einführungskatheter 9 ist in einem proximalen Endbereich zwischen den Stellen 26 und 27 mit einem Längsschlitz 28 versehen, durch den ein starres Rohrstück 29, das in der Bohrung 23 des Endstückes 22 befestigt ist, eingreift. Am proximalen Ende des Einführungskatheter 9 ist ein Griffstück 30 befestigt, das mit seiner konischen Außenfläche in einer konischen Öffnung des Endstückes 22 lösbar befestigt werden kann.

Die Handhabung der dargestellten Vorrichtung erfolgt auf die im folgenden beschriebene Weise.

Die dargestellte Vorrichtung wird ohne Sonde 7 in der dargestellten Lage vormontiert angeliefert. Die Sonde 7 ist vorzugsweise gesondert verpackt, z.B. in einem Feuchtbehälter, der das Austrocknen des Elektrolyten in einer Sonde vom Clark-Typ verhindern soll.

Es wird der Schädelknochen 4 des Patienten an einer Stelle freipräpariert und die Schädelbohrung 3 eingebracht. Die Dura 5 wird in der Mitte der Schädelbohrung 3 mit einem geeigneten Instrument, z.B. einer Lanzette, punktiert. Sodann wird die Schraube 1 zum Beispiel mittels eines auf einen Sechskantflansch 31 der Schraube 1 fassenden Schlüssels in die dargestellte Lage eingeschraubt.

Anstelle der Sonde 7 wird in die Vorrichtung ein nichtdargestellter Führungsdraht eingeführt, bis er genau in der dargestellten Lage der Sonde 7 liegt. Er wird am Endstück für die weitere Handhabung festgelegt, zum Beispiel verklammert.

Sodann wird die Vorrichtung an der Schraube 1 in der dargestellten Weise angesetzt unter Vorschieben des von dein Führungsdraht ausgesteiften Einführungskatheters 9 durch die zuvor punktierte Dura 5 bis in die dargestellte Lage im Gehirngewebe 6. Dabei wird die Hülse 13 mit ihrer Schiebeführung 14 auf den Ansatz 15 der Schraube 1 drehgesichert aufgesetzt und die Überwurfmutter 11 nur mit wenigen Drehungen lose auf dem Außengewinde 10 der Schraube 1 aufgeschraubt, zunächst ohne Quetschung der Quetschdichtung 16.

Nun wird der Führungsdraht herausgezogen und die Sonde bis in die dargestellte Lage eingeschoben. Sie wird dabei durch den Einführungskatheter 9 sicher an den gewünschten Ort gefühlt. Mit der Überwurfmutter 25 wird die Sonde 7 in ihrer achsialen Lage gesichert.

Sodann wird die Verbindung des Griffstückes 30 mit dem Endstück 22 gelöst und der Einführungskatheter 9 soweit zurückgezogen, bis sein distales Ende aus dein Gehirngewebe 6 herausgezogen ist, in dem dann nur noch die sehr dünne und flexible Sonde 7 liegt.

Anschließend wird durch weiteres Aufschrauben der Überwurfmutter 11 gegen die Schraube 1 die Quetschdichtung 16 komprimiert unter Herstellung der erwähnten Abdichtung.

Der elektrische Kupplungsstecker 24 wird an einem in der Nähe des Patienten aufgestellten, in der Zeichnung nicht dargestellten elektronischen Meßgerät angeschlossen und die Sonde 7 ist nun betriebsbereit.

Gegenüber der dargestellten bevorzugten Ausführungsform sind einige Varianten möglich.

So kann der Einführungskatheter 9 mit seinem proximalen Ende innerhalb des Schutzschlauches 20 noch vor dem Endstück 22 enden. Anstelle des Griffstückes 30 ist dann am proximalen Ende des Einführungskatheters 9 ein Griffstück vorzusehen, das durch einen Längsschlitz des Schutzschlauches 20 oder auf sonstige Weise durch diesen hindurch zur Längsverschiebung des Einführungskatheters betätigt werden kann. Das Rohrstück 29 im Endstück 22 kann dann entfallen.

Die Quetschdichtung kann auf andere als die dargestellte Weise betätigt werden, beispielsweise durch eine von außen angreifende Hebelpresse oder dergleichen.

Der Einführungskatheter 9 kann aus ausreichend eigensteifem Material sein, um auch ohne Einführungsdraht in das Gehirngewebe zielgenau verlegbar zu sein. Dann können sich gewisse Probleme mit der Abdichtung im Bereich der Quetschdichtung 16 zur Gewährleistung einer beschädigungsfreien Abdichtung auf der Sonde 7 ergeben. Diese können durch geeignete Ausbildung der Quetschichtung gelöst werden.

## Patentansprüche

1. Vorrichtung zum Halten und zur sterilen Abdichtung einer katheterförmigen Gehirnmeßsonde (7) in einer Bohrung (3) im Schädelknochen (4), mit einer mit einem distalen Außengewinde (2) in die Schädelbohrung (3) einschraubbaren Schraube (1) mit axialer Durchgangsbohrung (8), mit einem distal offenen Einführungskatheter (9) zur Aufnahme der Sonde (7), der in der Durchgangsbohrung (8) längsverschiebbar und mit einer Quetschdichtung (16) hermetisch und zugfest abdichtbar gelagert ist, mit einem zugfest über einen die Quetschdichtung (16) aufweisenden Schlauchanschluß (19) an der Schraube (1) befestigten, den Einführungskatheter (9) und die Sonde (7) umgebenden Schutzschlauch (20) und mit einem Kupplungsstecker (24) zum Anschluß des distalen Endes der Sonde (7), **dadurch gekennzeichnet**, daß der Einführungskatheter (9) als Schlauch aus einem bei Betätigung der Quetschdichtung (16) hermetisch auf der Sonde (7) abdichtenden und zugfest haltenden Material besteht, daß der Schutzschlauch (20) aus zugfestem Material besteht, an seinem proximalen Ende mit dem Kupplungsstecker (24) und an seinem distalen Ende mit der Schraube (1) zugfest verbindbar ist, daß der Einführungskatheter (9) innerhalb des Schutzschlauches (20) einen Längsschlitz (28) zur Einführung der Sonde (7) in den Einführungskatheter aufweist und daß eine distale Öffnung des Einführungskatheters (9) von der Sonde (7) axial durchfahrbar ausgebildet ist.

2. Vorrichtung nach Anspruch 1, **dadurch gekennzeichnet**, daß der Schlauchanschluß (19) eine mit einer proximal Befestigunseinrichtung für den Schutzschlauch versehene Schiebehülse (13) aufweist, die, von einer Spanneinrichtung (11) achsial gegen die Schraube (1) spannbar, drehgesichert gegenüber dieser verschiebbar ist unter Ausbildung eines ringförmig den Einführungskatheter (9) umgebenden, die ringförmige Quetschdichtung (16) aufnehmenden Quetschraumes, der außen von einer Umfangsfläche und in achsialer Richtung von Flächen (18, 17) der Hülse (13) und der Schraube (1) begrenzt ist.

3. Vorrichtung nach einem der vorhergehenden Ansprüche, **dadurch gekennzeichnet**, daß der Schutzschlauch (20) proximal an einem Endstück (22) befestigt ist, das lösbare Befestigungseinrichtungen (25) für den Kupplungsstecker (24) und Einrichtungen (30) zur achsialen Halterung des Einführungskatheters in seiner Einführungsstellung aufweist.

4. Vorrichtung nach einem der vorhergehenden Ansprüche, **dadurch gekennzeichnet**, daß ein anstelle der Sonde (7) während des Einführungsvorganges befestigt verlegbarer Führungsdraht vorgesehen ist.

5. Vorrichtung nach einem der vorhergehenden Ansprüche, **dadurch gekennzeichnet**, daß der Einführungskatheter (9) in einem proximalen Teil (26, 27) seiner Länge den Längsschlitz (28) aufweist und mit diesem über ein starres, am proximalen Ende des Schutzschlauches (20), die Sonde (7) umgebend befestigten Rohrstück (29) von der Sonde (7) seitlich abzieh- und auf diese aufschiebbar ist.

## Claims

1. Device for holding and for the sterile sealing of a brain scan probe (7) in the form of a catheter in a bore (3) in the skull, including a screw (1), which is screwable with a distal external screw thread (2) into the skull bore (3), with an axial passage bore (8), a distally open insertion catheter (9) for receiving the probe (7), which is longitudinally slidable in the passage bore (8) and may be sealed hermetically and in a tension-resistant manner with a pinch seal (16), a protective tube (20), which surrounds the insertion catheter (9) and the probe (7) and is secured in a tension-resistant manner to the screw (1) by means of a tube connection (9) affording the pinch seal and a coupling plug (24) for connecting the distal end of the probe (7), characterised in that the insertion catheter (9) comprises a tube of a tension-resistant material which seals hermetically on the probe (7) when the pinch seal (16) is actuated, that the protective tube (20) comprises tension-resistant material and may be connected in a tension-resistant manner at its proximal end to the coupling plug (24) and at its distal end to the screw (1), that the insertion catheter (9) has a longitudinal slot (28) within the protective tube (20) for introducing the probe (7) into the insertion catheter and that a distal opening of the insertion catheter (9) is constructed so that the probe (7) may move axially through it.

2. Device as claimed in claim 1, characterised in that the tube connector (19) has a sliding sleeve (13), which is provided with a proximal fastening device for the protective tube and may be clamped axially against the screw (1) by a clamping device (11) and is movable with respect to it whilst rotationally secured to form a pinch chamber, which annularly surrounds the insertion catheter (9) and accommodates the annular pinch seal (16) and which is defined externally by a peripheral surface and in the axial direction by surfaces (18,17) of the sleeve (13) and the screw (1).

3. Device as claimed in one of the preceding claims, characterised in that the protective tube (20) is fastened proximally to an end piece (22) which has releasable fastening devices (25) for the coupling plug (20) and devices (30) for axially retaining the insertion catheter in its inserted position.

4. Device as claimed in one of the preceding claims, characterised in that a guide wire is provided which may be fixed in position during the insertion process instead of the probe (7).

5. Device as claimed in one of the preceding claims, characterised in that the insertion catheter (9) affords a longitudinal slot (28) in a proximal portion (26,27) of its length and may be laterally pulled off from and pushed onto the probe (7) with it by means of a rigid tubular member (29) secured to the proximal end of the protective tube (20) so as to surround it.

## Revendications

1. Dispositif destiné à maintenir et étanchéifier de façon stérile une sonde de mesure cérébrale (7) en forme de cathéter dans un alésage (3) pratiqué dans l'os crânien (4), avec une vis (1) munie d'un filetage distal (2) et pouvant être vissée dans l'alésage crânien (3) et munie d'un perçage axial (8), avec un cathéter d'introduction (9) ouvert à son extrémité distale destiné à recevoir la sonde (7) et pouvant être déplacé longitudinalement dans le perçage (8) et y étant logé de façon hermétiquement étanche et résistante à la traction au moyen d'un joint à compression (16), avec un tuyau de protection (20) entourant le cathéter d'introduction (9) et la sonde (7) et fixé de façon résistante à la traction à la vis (1) par l'intermédiaire d'un raccord pour tuyau (19) présentant le joint à compression (16), et avec une fiche de couplage (24) destinée au branchement de l'extrémité distale de la sonde (7), **caractérisé en ce que** le cathéter d'introduction (9) est constitué, sous forme d'un tuyau, d'un matériau étanchéifiant de façon hermétique la sonde (7) et la maintenant de façon résistante à la traction lors de l'actionnement du joint à compression (16), et en ce que le tuyau de protection (20) est constitué d'un matériau résistant à la traction et est relié de façon résistante à la traction à son extrémité proximale à la fiche de couplage (24) et à son extrémité distale à la vis (1), et en ce que le cathéter d'introduction (9) présente, à l'intérieur du tuyau de protection (20), une fente longitudinale (28) destinée à l'introduction de la sonde (7) dans le cathéter d'introduction, et en ce qu'une ouverture distale du cathéter d'introduction (9) est conformée de façon à pouvoir être traversée axialement par la sonde (7).

2. Dispositif selon la revendication 1, **caractérisé en ce que** le raccord pour tuyau (19) présente une douille coulissante (13) munie d'un système proximal de fixation pour le tuyau de protection et qui. pouvant être serrée axialement sur la vis (1) à l'aide d'un mécanisme de serrage (11), peut coulisser relativement à la vis (1) en un mouvement assuré contre les torsions et formant un espace de compression entourant annulairement le cathéter d'introduction (9) et servant de logement au joint à compression (16) annulaire, lequel espace est délimité à l'extérieur par une surface périphérique et dans le sens axial par les surfaces (18, 17) de la douille (13) et de la vis (1).

3. Dispositif selon l'une des revendications précédentes, **caractérisé en ce que** le tuyau de protection (20) est fixé proximalement à un embout (22) qui présente des éléments amovibles de fixation (25) pour la fiche de couplage (24) et des éléments (3) pour le maintien axial du cathéter d'introduction en position d'introduction.

4. Dispositif selon l'une des revendications précédentes, **caractérisé en ce qu**'un fil guide pouvant être introduit et fixé est prévu en remplacement de la sonde (7) pendant le processus d'introduction.

5. Dispositif selon l'une des revendications précédentes, **caractérisé en ce que** le cathéter d'introduction (9) présente sur une partie proximale (26, 27) de sa longueur la fente longitudinale (28) et peut, grâce à celle-ci, être retiré latéralement de la sonde (7) et remis en place sur celle-ci par un tube (29) rigide fixé à l'extrémité proximale du tuyau de protection (20) de façon à entourer la sonde (7).
